# EUROPÄISCHE PATENTSCHRIFT

(11) **EP 0 511 572 B1**
(45) Veröffentlichungstag und Bekanntmachung des Hinweises auf die Patenterteilung: **28.06.1995**
(21) Anmeldenummer: 92106784.9
(22) Anmeldetag: 21.04.1992
(51) Int. Cl.: C07C 263/06, C07C 265/04, C08G 18/71, C07D 295/18

(54) **Neue Amin- und Ammoniumisocyanate, Verfahren zu ihrer Herstellung, ihre Verwendung zur Herstellung von Ammoniumgruppen enthaltenden Polymeren und die so hergestellten Polymere**
Amine and ammonium isocyanates, method of preparation, their use in preparing ammonium group containing polymers and the polymers thus prepared
Isocyanates d'amines et d'ammonium isocyanates, méthode pour leur préparation, leur utilisation pour la préparation des polymères contenant des groupes ammonium et polymères ainsi préparés

(30) Priorität: 01.05.1991 DE 4114221
(43) Veröffentlichungstag der Anmeldung: 04.11.1992
(73) Patentinhaber: Wolff Walsrode Aktiengesellschaft, D-29655 Walsrode (DE)
(72) Erfinder: Buysch, Hans-Josef, Dr., W-4150 Krefeld (DE); König, Klaus, Dr., W-5068 Odenthal (DE); Klausener, Alexander, Dr., W-4150 Krefeld (DE); Szablikowski, Klaus, Dr., W-3030 Walsrode (DE); Breckwoldt, Jörn, Dr., W-2720 Rotenburg (DE)
(74) Vertreter: Zobel, Manfred, Dr.

(56) Entgegenhaltungen:
- EP-A- 0 001 603
- EP-A- 0 131 859
- BE-A- 631 961
- GB-A- 890 519
- JP-A-48 103 582
- CHEMICAL ABSTRACTS, vol. 80, no. 17, 29. April 1974, Columbus, Ohio, US;abstract no. 95738, M INOUE ET AL: 'Pyridine derivatives' Seite 394 ;Spalte 1 ;

## Beschreibung

Die vorliegende Erfindung betrifft neue Ammoniumisocyanate der Formel I,

Y-R-NCO (I)

worin
- R: eine C₂-C₁₀-Alkylengruppe,
- Y: eine Ammoniumgruppe 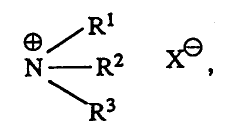
R¹, R², R³ gleich oder verschieden sind und C₁-C₅-Alkyl, R¹ und R² zusammen mit dem N-Atom zusätzlich einen gegebenenfalls ein O-Atom enthaltenden 5- oder 6-Ring,
- R³: zusätzlich Allyl, Methallyl und Benzyl,
- X^{⊖}: ein Anion einer starken Säure darstellen,
ein Verfahren zu ihrer Herstellung durch Umsetzung von Harnstoffen der Formel II 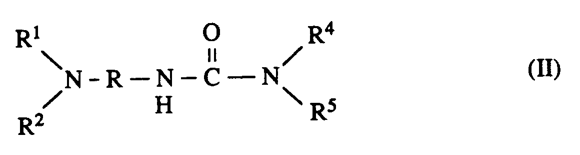
mit R⁴ und R⁵ gleich oder verschieden und C₁₋₅-Alkyl oder R⁴ und R⁵ zusammen mit dem N-Atom einen gegebenenfalls durch ein O-Atom unterbrochenen 5- oder 6-gliedrigen carbocyclischen Ring mit Mono- oder Polyisocyanaten bei Temperaturen von 50 bis 250°C und Drucken von 0,001 bis 1 bar unter Abdestillieren der Aminoisocyanate der Formel III, 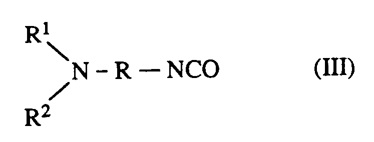
deren Alkylierung mit Verbindungen der Formel R³ X zu den Ammoniumisocyanaten IV 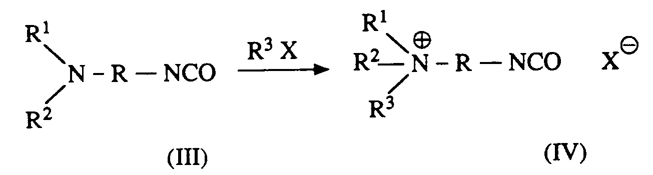
und schließlich Ammoniumgruppen enthaltende Polymere durch Umsetzung von OH-Gruppen enthaltenden Polymeren mit den Isocyanaten der Formel IV.

Es ist bekannt, Harnstoffe von niederen Aminen in Gegenwart hochsiedender Isocyanate in niedriger siedende Isocyanate thermisch zu spalten und das niedrig siedende Isocyanat aus dem Reaktionsgemisch zu entfernen. Die Reaktion läuft nach folgendem Schema ab: 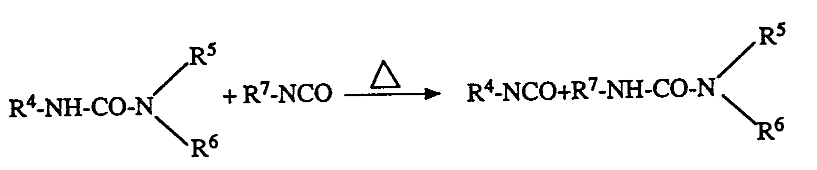
Diese Umsetzung ist in der EP-A 001 603 eingehend beschrieben.

Es wurde nun überraschend gefunden, daß auf diesem Wege auch Isocyanate mit tert. Aminogruppen zuganglich sind. Dies ist umso überraschender als tert. Amine die Oligomerisierung von Isocyanaten beispielsweise zu Isocyanuraten katalysieren, insbesondere bei erhöhter Temperatur. Bei der vorliegenden hohen Aminkonzentration und der hohen Spaltungstemperatur mußte demnach mit einer raschen und vollständigen Umwandlung der neuen Aminoisocyanate in höhermolekulare Produkte gerechnet werden.

Gegenstand der Erfindung ist weiter ein Verfahren zur Herstellung von Ammoniumisocyanaten, dadurch gekennzeichnet, daß das Verfahren in folgenden Schritten durchgeführt wird:
a) Reaktion von Kohlensäurederivaten mit primär-tertiären Diaminen der Formel IIa unter Ausbildung eines Urethanderivates.

Geeignete Ausgangsprodukte für das erfindungsgemäße Verfahren sind primär-tertiäre Diamine der Formel IIa, Worin R eine C₂-C₁₀-Alkylengruppe darstellt, die linear oder verzweigt sein kann, vorzugsweise C₂ bis C₈-Alkylen, besonders bevorzugt C₂ bis C₆-Alkylen; R¹ und R² C₁ bis C₅-Alkyl oder zusammen mit dem N-Atom einen 5- oder 6-Ring, der gegebenenfalls ein O-Atom enthalten kann, bevorzugt C₁-C₄-Alkyl, -(CH₂)₄, -(CH₂)₅, -(CH₂CH₂)₂O, besonders bevorzugt CH₃, C₂H₅, (CH₂)₄, (CH₂)₅ und -(CH₂CH₂)₂O. 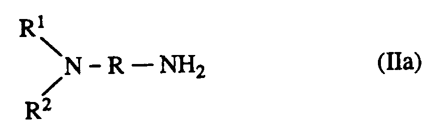
Genannt seien beispielsweise 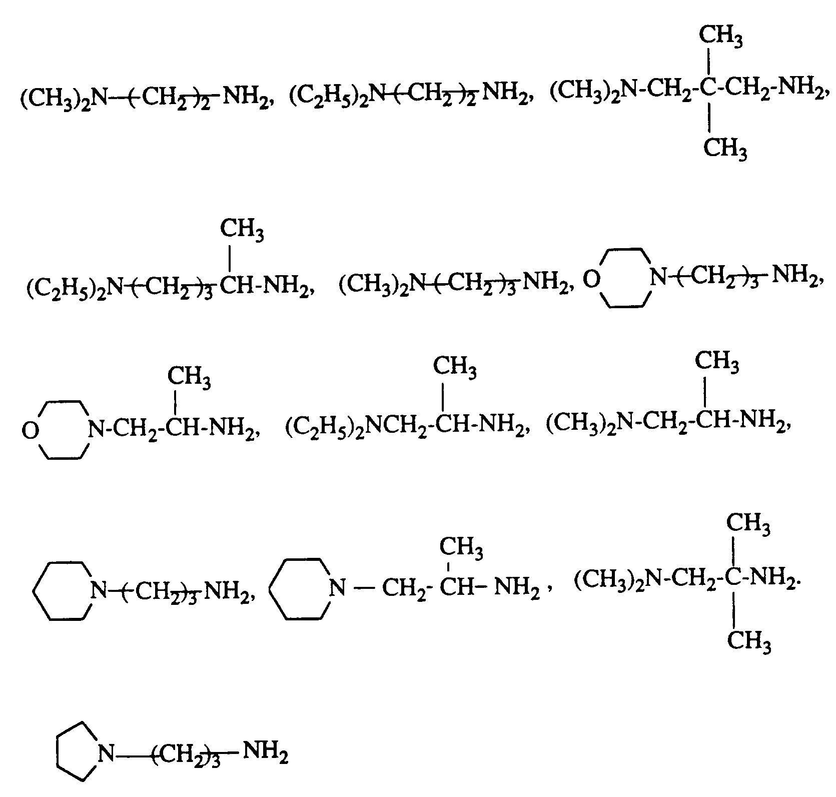
b) Umsetzung des Urethanderivats mit sekundären Aminen der Formel 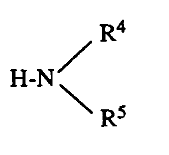 zum Harnstoffderivat der Formel II, wobei die Substituenten R⁴, R⁵ die oben angegebene Bedeutung besitzen.
c) Thermische Spaltung der Harnstoffe in Gegenwart hochsiedender Isocyanate unter Abdestillieren der Aminoisocyanate der Formel III.

Geeignete Ausgangsisocyanate sind ausführlich in der EP-A 001 603 beschrieben, desgleichen die Verfahrensweisen auf den S. 5 bis 8. Die Durchführung des Verfahrens in Gegenwart von Carbodiimiden ist jedoch im vorliegenden Fall nicht erforderlich. Für die Herstellung der Aminoisocyanate sind die genannten aliphatischen Isocyanate besonders bevorzugt.

Die auf diese Weise erhaltenen Aminoisocyanate der Formel III werden mit Alkylierungsmitteln der Formel R³X in quartäre Verbindungen übergeführt, dadurch, daß man entweder das Aminoisocyanat in das Alkylierungsmittel oder umgekehrt, das Alkylierungsmittel in das Aminoisocyanat oder beide gleichzeitig in das Reaktionsgefäß einträgt. Man kann die Reaktion in Substanz oder in geeigneten inerten Lösungsmitteln durchführen. Geeignet sind z.B. Kohlenwasserstoffe wie Hexan, Pentan, Cyclohexan, Benzol, Toluol, Xylol, Halogenkohlenwasserstoffe wie Dichlormethan, Trichlormethan, Dichlorethan, Trichlorethan, Chlorbenzol, Brombenzol, Dichlorbenzol, Ether wie Diethyl-, Diisopropylether, Anisol, Dibutylether, Nitrile wie Acetonitril, Propionitril und Benzonitril. Man kann auch das Alkylierungsmittel als Verdünnungsmittel verwenden, wenn es leicht wiedergewonnen werden kann, z.B. Methylchlorid, Ethylchlorid, Methyliodid oder Propylbromid. Die Umsetzung wird bei 0 bis 100°C, vorzugsweise 10 bis 80°C, durchführt.

Geeignete Alkylierungsmittel R³X sind solche mit R³ C₁-C₅-Alkyl, Allyl, Methallyl oder Benzyl, bevorzugt sind C₁-C₄-Alkyl, Allyl und Benzyl, besonders bevorzugt Methyl, Ethyl, Allyl und Benzyl.
X ist der Rest einer starken Säure. Das sind solche, die einen in Wasser gemessenen pkₐ-Wert kleiner als 3, bevorzugt Kleiner als 2 besitzen.

Genannt seien z.B. die Halogenwasserstoffsäuren HCl, HBr, HJ, Schwefelsäure, Phosphorsäure, Sulfonsäuren wie Benzol-, Chlorbenzol- und Toluolsulfonsäure und Phosphonsäuren wie Chlorbenzol- oder Cyanbenzolphosphonsäure.

Die auf diese Weise hergestellten Ammoniumisocyanate sind neu und daher Gegenstand vorliegender Erfindung. Sie sind wertvolle Ausgangsprodukte zur Herstellung von Polymeren mit quartären Ammoniumgruppen, die dadurch erhalten werden, daß man OH-haltige Polymere mit diesen Ammoniumisocyanaten in Lösung oder Suspension umsetzt.

Geeignete OH-gruppenhaltige Polymere sind z.B. Polyvinylalkohole, Polyacrylate, die unter Verwendung von OH-Gruppen tragenden Monomeren wie β-Hydroxyethyl(meth)acrylat hergestellt wurden, besonders aber Polysaccharide, Starke, Dextrine, Glycogen, Polyglucosane wie Cellulose und deren Derivate, z.B. Methylcellulose, Hydroxyethylcellulose, Hydroxypropylcellulose, gemischte Celluloseether wie Methylhydroxyethylcellulose, Methylhydroxypropylcellulose, Hydroxyethylhydroxypropylcellulose, Sulfoethylcellulose, Sulfoethylcarboxymethylcellulose, Methylsulfoethylcellulose, Hydroxyethylsulfoethylcellulose, Dihydroxypropylcellulose, Dihydroxypropylhydroxyethylcellulose, Dihydroxypropylcarboxymethylcellulose, Carboxymethylcellulose, deren Ester und Salze mit Natrium-, Kalium-, Calcium- und Ammoniumionen, Carboxymethylhydroxyethylcellulose, Cellulosesulfat, Polyfructosane wie Inulin und Graninin, Polymannosane, Polygalactosane, auch gemischte Polysaccharide wie Hemicellulosen, ferner Polyxylosane, Polyarabiosane sowie auch Heteropolysaccharide wie Gellan, Xanthan und Pullulan.

Bevorzugt sind Cellulose und ihre Derivate, Starke und Dextrine, besonders bevorzugt Cellulose, Methylcellulose, Hydroxyethylcellulose, Hydroxypropylcellulose und Starke.

Quartäre Ammoniumpruppen enthaltende kationische Polymere, besonders kationische Polysaccharide sind wichtige Produkte und Hilfsmittel für die Herstellung von Papier, medizinischen Filtern, kosmetischen Reinigungsmitteln und Flockungsmitteln für die Klärung von Abwässern.

Die Herstellung solcher Produkte ist bisher unbefriedigend gelöst. Mehrere Verfahren sind vorgeschlagen worden, die in der Regel aber nur zu niedrigen Substitutionsgraden an den Polysacchariden führen. Nach der EP-A 367 003 (siehe auch dort zit. Lit.) ist es jedoch möglich, hochsubstituierte Polysaccharide zu erhalten. Das dort beschriebene Verfahren erfordert jedoch drei nacheinander zu vollziehende polymeranaloge Umsetzungen am Polysaccharid und damit entsprechenden Zeit- und Aufarbeitungsaufwand.

Es wurde nun gefunden, daß die oben beschriebenen Ammoniumisocyanate sich sehr einfach mit Polysacchariden umsetzen lassen und in einem einzigen polymeranalogen Reaktionsschritt kationische Polysaccharide, ergeben, die den in der EP-A 367 003 beschriebenen vergleichbar sind. Darum ist auch die Verwendung der erfindungsgemäßen Isocyanate der Formel I zur Herstellung von kationischen Polymeren, besonders kationischen Polysacchariden, und die nach diesem Verfahren hergestellten kationischen Polymere, besonders kationische Polysaccharide, Gegenstand der vorliegenden Erfindung.

Die Umsetzung der Ammoniumisocyanate IV mit den OH-gruppenhaltigen Polymeren erfolgt in Lösung oder Dispersion, wobei die Polysaccharide bevorzugt in Suspension eingesetzt werden, weil sie sehr hochviskose Lösungen bilden, die nur geringe Feststoffgehalte erlauben und damit großen Reaktionsraum und umständliche Aufarbeitung erfordern.

Geeignete Lösungs- und Dispersionsmittel sind inert unter den Reaktionsbedingungen. Genannt seien beispielsweise Kohlenwasserstoffe wie Cyclohexan, Pentan, Heptan, Isooctan, Benzol, Toluol, Halogenkohlenwasserstoffe wie Methylenchlorid, Chloroform, Dichlorethan, Trichlorethylen, Ether wie Diethyl-, Diisopropyl-, Dibutylether, Dioxan, Anisol, Dimethoxyethan, Ester wie Essigsäureethylester, -butylester, Propionsäureethylester, Benzoesäureethylester, Ketone wie Aceton, Methylethylketon, Diethylketon, Methylisopropylketon, Cyclohexanon, Acetophenon, Amide wie Dimethylformamid, Dimethylacetamid, Tetramethylharnstoff, N,N-Dimethylethylenharnstoff, N-Methylpyrrolidon, N-Methylcaprolactam, Nitrile wie Acetonitril, Propionitril, Benzonitril, β-Methoxypropionitril und β-Cyano-β'-methoxy-diethylether.

Die Reaktionstemperatur liegt bei 0 bis 120°C, vorzugsweise 10 bis 100°C, bevorzugt 15 bis 80°C.

Die Ausgangsprodukte und Lösungs- bzw. Dispersionsmittel sollten im wesentlichen wasserfrei sein, damit möglichst wenig Nebenreaktionen des Ammoniumisocyanats mit Wasser erfolgen.

Das OH-gruppenhaltige Polymer wird in der Regel vorgelegt im entsprechenden Medium und das Ammoniumisocyanat als solches oder auch in dem Lösungsmittel gelöst, in dem auch die Alkylierung erfolgte, zudosiert, wobei sowohl für die Alkylierung wie polymeranaloge Umsetzung ein und dasselbe Medium verwendet werden kann. Die Reaktionszeit beträgt zwischen 30 min und mehreren Stunden.

Die Isocyanataddition an die OH-funktionellen Polymere verläuft in der Regel langsam, so daß sie zweckmäßig katalysiert wird. Als Katalysatoren werden die bei der Urethanbildung aus Isocyanaten und Alkoholen üblicherweise verwendeten Katalysatoren in den vorgeschlagenen Mengen (siehe Houben-Weyl, Bd. E20, 1604 ff (1987)) eingesetzt. Besonders bevorzugte Katalysatoren sind organische Zinnverbindungen wie Dibutylzinndilaurat oder Zinndilaurat.

Der Gegenstand der Erfindung soll anhand der folgenden Beispiele näher erläutert werden. %-Angaben sind - soweit nicht anders angegeben - Gew.-%.

### Beispiel 1

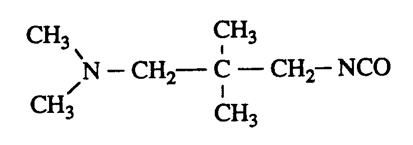

381 g (1,78 Mol) Diphenylcarbonat werden in 200 ml 1,2-Dichlorethan aufgeschlämmt und unter Eiskühlung bei 0 bis 10°C 232 g (1,78 Mol) Dimethylaminoneopentylamin zugetropft (Dauer 60 min). Nach 2 Stdn. bei 20°C ist die Reaktion zum O-Phenylurethan beendet (Titration mit HCl). Nun werden 130 g (1,78 Mol) Diethylamin unter Kaltwasserkühlung zugetropft und anschließend kurz auf 100°C bis zum vollständigen Umsatz des Urethans zum trisubstituierten Harnstoff. 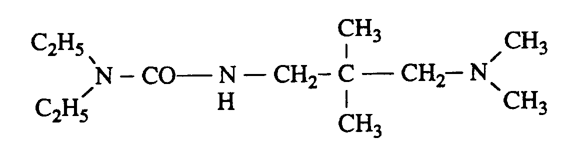
Nun wird beginnend bei 200 mm und zuletzt bei 1 mm zunächst Dichlorethan und dann Phenol abdestilliert, der flüssige Rückstand bei 60°C mit 450 g Hexamethylendiisocyanat (2,68 Mol) versetzt und bei 15 mbar hochgeheizt. Ab 100°C im Sumpf beginnt die Destillation bis 150°C. Dabei werden in 2 Stdn. 245 g (88,4 % der Theorie), Kp₁₅ 70°C, der Titelverbindung als leicht bewegliche, wasserklare, übelriechende Flüssigkeit erhalten.

### Beispiel 2

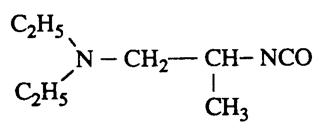

Das Ausgangsamin wird analog Beispiel 1 in Methylenchlorid mit Diphenylcarbonat und dann mit Diethylamin zum Harnstoff und schließlich mit Hexamethylendiisocyanat in die Titelverbindung übergeführt:
Ausbeute: 85 % der Theorie, Kp 30°C 0,4 mbar, 72°C 13 mbar,
99 %ig nach gaschromatographischer Analyse.

### Beispiel 3

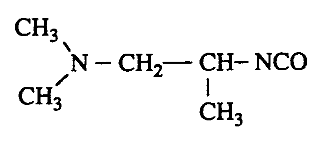

Diese Verbindung wird analog Beispiel 1 hergestellt, wobei jedoch statt Hexamethylendiisocyanat Isophorondiisocyanat verwendet wird:
Ausbeute: 40 % Kp 70°C/50 mbar
Fp 59°C; NCO: 98,5 % der Theorie.

### Beispiel 4

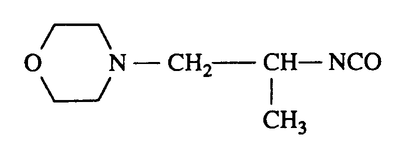

Herstellung analog Beispiel 3.
Ausbeute: 75 %, Kp 0,5 mbar 50°C, 99,3 %ig nach gaschromatographischer Analyse.

### Beispiel 5

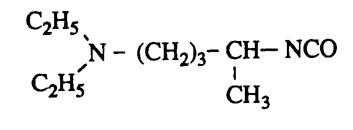

Herstellung analog Beispiel 3.
Ausbeute: 82 % der Theorie, Kp 0,3 mbar 48°C, 100 %ig nach gaschromatographischer Analyse.

### Beispiel 6

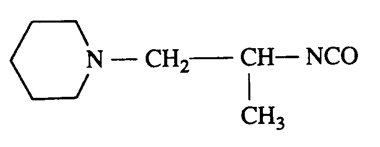

Herstellung analog Beispiel 3.
Ausbeute: 79 % der Theorie, Kp 0,4 mbar 42°C, 99 %ig nach gaschromatographischer Analyse.

### Beispiel 7

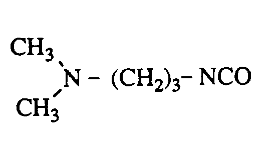

Herstellung nach Beispiel 3.
Ausbeute: 71 % der Theorie, Kp 13 mbar 57°C, 99,8 %ig nach gaschromatographischer Analyse.

### Beispiel 8

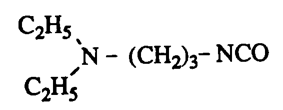

Herstellung analog Beispiel 3.
Ausbeute: 79 % der Theorie, Kp 0,2 mbar 26°C, 98 %ig nach gaschromatographrscher Analyse.

### Beispiel 9

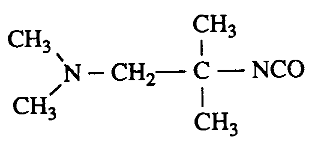

Herstellung nach Beispiel 3.
Ausbeute: 50 % der Theorie, Kp 13 mbar 47°C.

### Beispiel 10

128 g (1,0 Mol) des Destillats aus Beispiel 7 wird in 200 ml wasserfreiem Methylenchlorid gelöst und innerhalb einer Stunde zu einer wasserfreien siedenden Lösung von 130 g (1,02 Mol) Dimethylsulfat in 300 ml Methylenchlorid hinzugetropft Man hält noch 1 h unter Rückfluß. Nach Abdestillieren von Methylenchlorid im Vakuum erhält man ein gelbliches Öl, das nach Analyse der Formel entspricht: 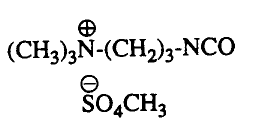
Auf diese Weise werden andere Ammoniumisocyanate aus den Aminoisocyanaten der Beispiele 2 bis 9 hergestellt.

### Beispiel 11

90 g (0,51 Mol bezüglich Methylcelluloseeinheit) einer Methylcellulose mit einem Substitutionsgrad DS von 1 (DS=Anzahl der Substituenten pro Celluloseeinheit) wird in 1000 ml trockenem Methylenchlorid suspendiert und durch azeotrope Destillation entwässert. Man erhält eine gut rührbare Suspension in ca. 500 ml Methylenchlorid. Dazu gibt man 40 g wasserfreies Dimethylacetamid, 0,1 g Dibutylzinndilaurat und 3/4 des Reaktionsproduktes aus Beispiel 10 entsprechend 0,75 Mol an Ammoniumisocyanat. Das Gemisch wird unter Rückfluß gekocht und an Proben der Fortschritt der Isocyanataddition gemessen. Nach 20 Stdn. wird die Reaktion abgebrochen, die umgesetzte Cellulose abgesaugt, dreimal gründlich mit Methylenchlorid, viermal mit Isopropanol gewaschen und im Vakuum bei 50°C getrocknet. Man erhält 215 g einer kationischen Cellulose, die im IR-Spektrum eine starke Urethanbande bei 1725 cm⁻¹ zeigt, einen N-Gehalt von 7,8 % und einen S-Gehalt von 9,7 % aufweist. Dies entspricht einem Substitutionsgrad DS von etwa 1,3.

## Patentansprüche

1. Ammoniumisocyanate der allgemeinen Formel 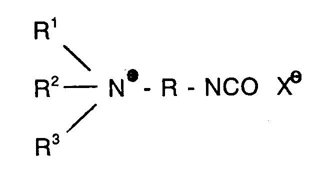 worin
R eine geradkettige oder verzweigte Alkylengruppe mit 2 bis 10 C-Atomen,
R¹, R², R³ gleich oder verschieden sind und Alkylgruppen mit 1 bis 5 C-Atomen bedeuten,
R¹ und R² zusammen mit dem N-Atom außerdem zusammen mit 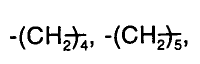 oder (̵CH₂CH₂)O einen Ring darstellt,
R³ außerdem Allyl, Methallyl und Benzyl und
X^{⊖} ein Anion starker Säuren darstellt.

2. Verfahren zur Herstellung von Ammoniumisocyanaten der Formel 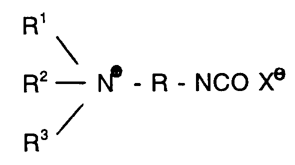 worin R¹, R² und R³ die oben angegebene Bedeutung haben, durch Umsetzung von Harnstoffen mit Isocyanaten und anschließender thermischer Spaltung, wobei man als Harnstoffe solche der Formel II einsetzt, 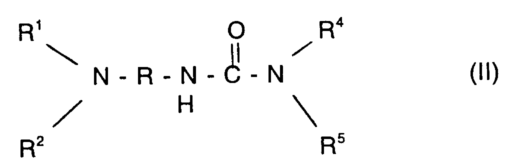 worin R, R¹, R² die oben angegebene Bedeutung und R⁴ und R⁵ die Bedeutung von R¹ und R² haben, dadurch gekennzeichnet, daß man die erhaltenen Aminoisocyanate 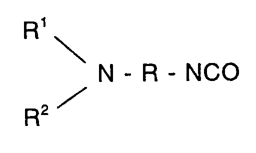 durch Alkylierung mit Verbindungen der Formel R³X zu Ammoniumisocyanaten umsetzt, worin R³ die o.a. Bedeutung hat.

3. Verwendung der Ammoniumisocyanate gemäß Anspruch 1 zur Herstellung von Polymeren mit quartären Ammoniumgruppen, dadurch gekennzeichnet, daß man OH-gruppenhaltige Polymere mit Ammoniumisocyanaten gemäß Anspruch 1 umsetzt.

4. Verfahren zur Herstellung von kationischen Polymeren mit quartären Ammoniumgruppen durch Umsetzung von OH-funktionellen Polymeren mit Ammoniumisocyanaten, dadurch gekennzeichnet, daß man Ammoniumisocyanate gemäß Anspruch 1 einsetzt.

## Claims

1. Ammonium isocyanates of the general formula 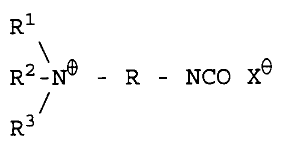 in which
R means a straight or branched alkylene group with 2 to 10 C atoms,
R¹, R², R³ are identical or different and mean alkylene groups with 1 to 5 C atoms,
R¹ and R² together with the N atom moreover denote a ring together with -(CH₂-)₄-, -(CH₂-)₅- or -(-CH₂CH₂)O,
R³ moreover denotes allyl, methallyl and benzyl and
X^{⊖} denotes an anion of a strong acid.

2. Process for the production of ammonium isocyanates of the formula 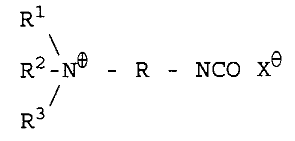 in which R¹, R² and R³ have the above-stated meaning by the reaction of ureas with isocyanates and subsequent thermal decomposition, wherein the ureas used are those of the formula II, 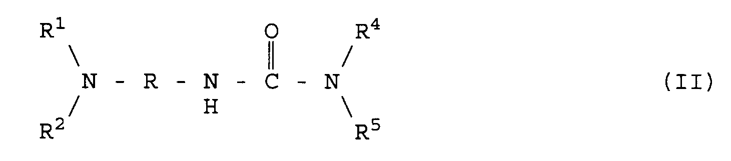 in which R, R¹, R² have the above-stated meaning and R⁴ and R⁵ have the meaning of R¹ and R², characterised in that the aminoisocyanates obtained 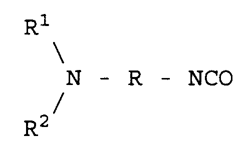 are alkylated with compounds of the formula R³X to yield ammonium isocyanates, in which R³ has the above-stated meaning.

3. Use of the ammonium isocyanates according to claim 1 for the production of polymers with quaternary ammonium groups, characterised in that polymers containing OH groups are reacted with ammonium isocyanates according to claim 1.

4. Process for the production of cationic polymers with quaternary ammonium groups by the reaction of OH-functional polymers with ammonium isocyanates, characterised in that ammonium isocyanates according to claim 1 are used.

## Revendications

1. Isocyanates d'ammonium répondant à la formule générale 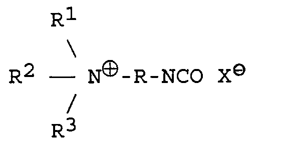 dans laquelle
R représente un groupe alkylène à chaîne droite ou ramifiée contenant de 2 à 10 atomes de carbone,
R¹, R², R³ sont identiques ou différents et représentent des groupes alkyle contenant de 1 à 5 atomes de carbone,
R¹ et R², ensemble avec l'atome N, en outre ensemble avec 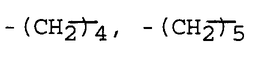 ou (̵CH₂CH₂)₂O, représentent un cycle,
R³ représente, en outre, un groupe allyle, un groupe méthallyle et un groupe benzyle et
X^{⊖} représente un anion d'acides forts.

2. Procédé pour la préparation d'isocyanates d'ammonium répondant à la formule 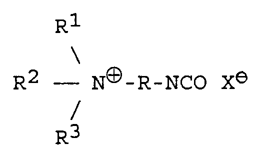 dans laquelle R¹, R² et R³ ont la signification indiquée ci-dessus, par mise en réaction d'urées avec des isocyanates et par scission thermique ultérieure, dans lequel on utilise, comme urées, celles répondant à la formule II 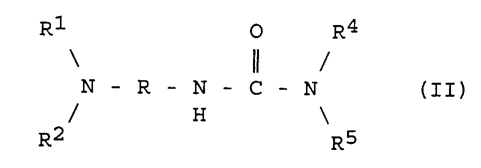 dans laquelle R, R¹, R² ont la signification indiquée ci-dessus et R⁴ et R⁵ ont la signification donnée pour R¹ et R², caractérisé en ce qu'on fait réagir les aminoisocyanates obtenus 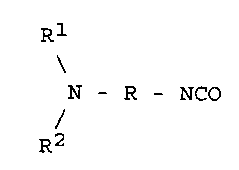 par alkylation avec des composés de formule R³X pour obtenir des isocyanates d'ammonium, dans laquelle R³ a la signification indiquée ci-dessus.

3. Utilisation des isocyanates d'ammonium selon la revendication 1 pour la préparation de polymères contenant des groupes d'ammonium quaternaires, caractérisée en ce qu'on fait réagir des polymères contenant des groupes OH avec des isocyanates d'ammonium selon la revendication 1.

4. Procédé pour la préparation de polymères cationiques contenant des groupes d'ammonium quaternaires par mise en réaction de polymères OH-fonctionnels avec des isocyanates d'ammonium, caractérisé en ce qu'on met en oeuvre des isocyanates d'ammonium selon la revendication 1.
